# DEMANDE DE BREVET EUROPEEN

(11) **EP 0 670 150 A1**
(43) Date de publication de la demande: **06.09.1995**
(21) Numéro de dépôt: 95400209.3
(22) Date de dépôt: 01.02.1995
(51) Int. Cl.: A61F 2/01

(54) **Filtre sanguin endovasculaire à stabilisation axiale**

(30) Priorité: 07.02.1994 FR 9401344
(71) Demandeur: B. BRAUN CELSA, Société Anonyme, F-86360 Chasseneuil du Poitou (FR)
(72) Inventeur: Bovyn, Gilles, F-22000 Saint-Brieuc (FR); Chevillon, Gérard, F-92120 Montrouge (FR); Cottenceau, Jean-Philippe, F-92160 Antony (FR)
(74) Mandataire: Lerner, François

(57) **Abrégé**

Il s'agit d'un dispositif à usage médical propre à être introduit dans un conduit d'un corps humain ou animal vivant, en particulier pour une utilisation comme filtre sanguin endovasculaire, le dispositif présentant un axe (10) et comprenant une tige flexible (2) qui présente une extrémité proximale (2a), une extrémité distale (2b) et une longueur (L) suivant l'axe du dispositif.

Selon l'invention, ce dispositif présente localement, sur une partie de la longueur de la tige, au moins une zone courbée à courbure déformable de stabilisation axiale du dispositif, cette zone étant propre à varier de courbure lors d'un mouvement axial de l'une desdites extrémités de la tige par rapport à l'autre.

## Description

L'invention se rapporte au domaine des dispositifs à usage médical implantables dans un conduit d'un corps humain ou animal vivant.

Dans ce cadre général, l'invention concerne en particulier les dispositifs "vasculaires" qui comprennent une tige flexible allongée, éventuellement creuse, introductible dans un vaisseau.

Tout particulièrement, l'invention se rapporte à des dispositifs de filtration sanguine dont la tige (ou cathéther) est liée, à une extrémité, à un filtre sanguin. L'implantation de ce filtre s'effectue, en général, dans la veine cave inférieure arrivant au coeur. On peut ainsi arrêter avant leur entrée dans le coeur d'éventuels caillots de sang risquant d'entraîner des embolies.

Parmi ces unités de filtration "in situ", on en connaît qui sont dites "temporaires" parce qu'elles sont plus particulièrement implantées pour une période de temps limitée. En effet, l'un des avantages de ce type de dispositif est que normalement le praticien peut, lorsqu'il le désire, utiliser la tige porteuse pour retirer la prothèse vasculaire qui est normalement dépourvue de moyens d'ancrage à la paroi du vaisseau.

L'expérience a cependant montré qu'il pouvait se poser dans certaines conditions des problèmes de migration de tout ou partie du dispositif, dûs aux contraintes mécaniques exercées par le milieu environnant, contraintes qui s'appliquent a priori essentiellement sur la prothèse vasculaire. Ainsi, on a constaté qu'une tige souple, favorable au glissement du dispositif le long de la voie d'accès, tendait parfois à amortir la poussée exercée par la prothèse en formant des boucles, avec pour conséquence un déplacement progressif de cette prothèse. Dans le cas des filtres sanguins, on a parfois observé une remontée du filtre vers le coeur, avec les risques inhérents que l'on imagine.

On a également noté qu'une tige plus rigide risquait de ne plus amortir la poussée exercée sur elle par la prothèse, la tige pouvant alors avoir tendance à tirer sur les tissus.

Face à cela, l'invention propose tout d'abord un dispositif présentant comme axe celui de la tige et se caractérisant en ce qu'il présente localement, sur une partie de la longueur de cette tige, au moins une zone courbée à courbure déformable de stabilisation axiale du dispositif, ladite zone étant propre à varier de courbure lors d'un mouvement axial de l'une des extrémités de la tige par rapport à l'autre.

Cette zone de stabilisation permet de maintenir correctement en place l'ensemble du dispositif puisqu'elle doit se comporter comme un "ressort" qui amortit les forces essentiellement axiales pouvant lui être communiquées.

Dans le domaine de la filtration sanguine, la solution proposée a notamment pour avantage de permettre un positionnement précis et sûr du dispositif, la zone précitée de stabilisation pouvant tout particulièrement être disposée dans l'oreillette droite du coeur où elle se maintiendra.

Comme on le verra ci-après, la zone courbée pourra soit être formée par une portion de la tige elle-même, soit comprendre une pièce annexe au moins localement courbée et disposée le long de la tige. Dans le second cas, la pièce annexe sera avantageusement disposée coulissante, soit à l'intérieur de la tige (qui sera alors creuse), soit autour d'elle, pour faire varier à volonté la position axiale de la zone courbée.

En alternative, le dispositif pourrait également comporter une gaine extérieure tubulaire entourant la tige avec un certain jeu, la pièce courbée de maintien étant alors disposée entre cette gaine et la tige.

A titre complémentaire, l'invention se rapporte en outre à la tige en elle-même, en ce qu'elle présente, sur une partie de sa longueur et dans un état de repos, au moins une zone courbée à courbure déformable, cette zone étant sensiblement élastiquement déformable en flexion pour reprendre sensiblement sa forme, après qu'on ait poussé ou tiré sur elle suivant l'axe de la tige.

L'invention se rapporte également, en alternative, à la pièce rapportée de maintien en forme d'une tige ou d'un cathéter "classique", cette pièce présentant naturellement ladite zone courbée élastiquement flexible, de manière à pouvoir reprendre sensiblement sa forme après déformation élastique axiale.

Outre le dispositif précité en tant que tel, l'invention se rapporte encore à un procédé de réalisation d'un tel dispositif, dans lequel :
a) on utilise une tige biocompatible, sensiblement élastiquement déformable en flexion,
b) et on donne une forme courbée à une portion de cette tige, en s'assurant que cette portion reprend sensiblement sa forme courbée après qu'on ait poussé ou tiré sur elle suivant l'axe d'allongement de la tige.

A toutes fins utiles, on notera que l'extrémité "distale" des éléments du dispositif désigne l'extrémité qui doit être implantée le plus profondément dans le corps du patient, l'extrémité "proximale" étant l'extrémité opposée située près de la surface de la peau (voire à l'extérieur).

D'autres caractéristiques et avantages de l'invention apparaîtront encore de la description qui va suivre, faite en référence aux dessins annexés, dans lesquels :
- la figure 1 est une vue en perspective du dispositif selon l'invention, pourvu d'une prothèse vasculaire et représenté dans un état de repos,
- la figure 2 est une vue en coupe schématique du corps d'un patient dans lequel est implanté le dispositif de la figure 1,
- la figure 3 montre en vue agrandie en perspective une partie du dispositif de la figure 1,
- les figures 4 et 5 sont des variantes de réalisation de la zone courbée de la tige de la figure 1,
- les figures 6 et 7 montrent en vue en coupe, partielle et agrandie, des variantes de réalisation du dispositif de la figure 1,
- les figures 8 et 9 montrent certains des principaux moyens utilisés pour la mise en place ou le retrait du dispostif de l'invention, et
- la figure 10 montre en coupe une autre variante du dispositif de la figure 1.

L'un des intérêts de l'utilisation de ce dispositif étant de pouvoir améliorer les conditions d'implantation des filtres sanguins temporaires, on ne décrira ci-après l'invention que dans le cadre d'une telle application, même s'il doit être clair qu'elle peut s'appliquer à d'autres "prothèses médicales", même non vasculaires.

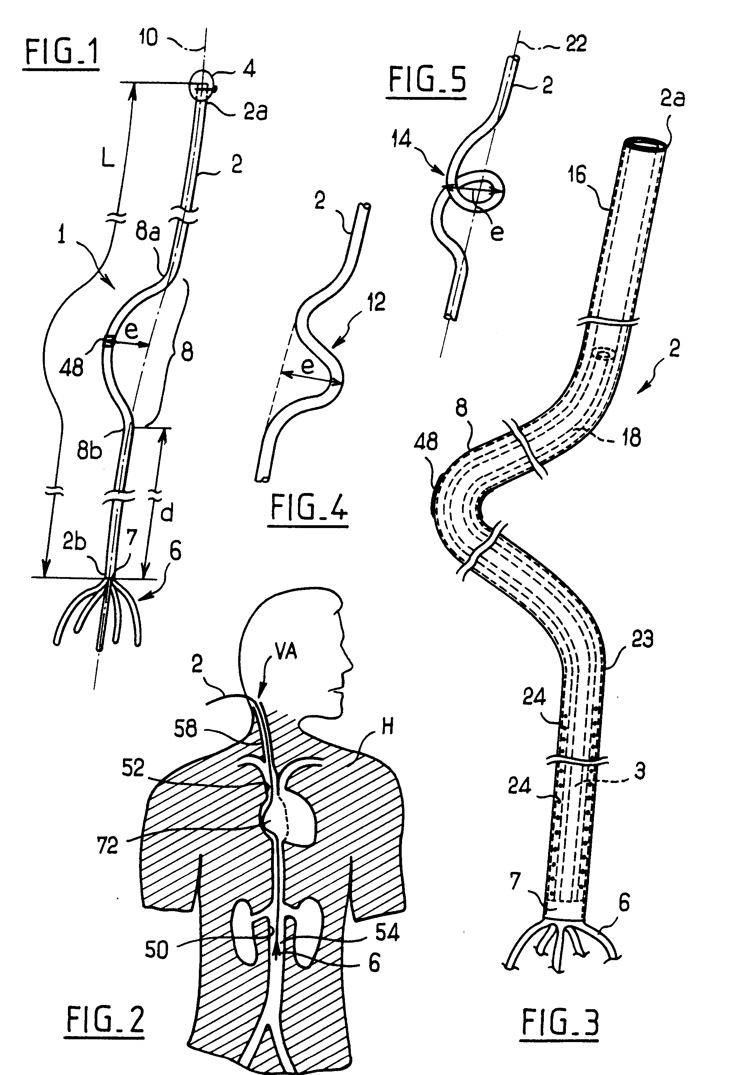

Sur la figure 1, on voit donc illustrée dans son ensemble une unité temporaire de filtration sanguine 1, d'axe général sensiblement rectiligne 10, pouvant être mise en place par voie percutanée, ou par dénudation.

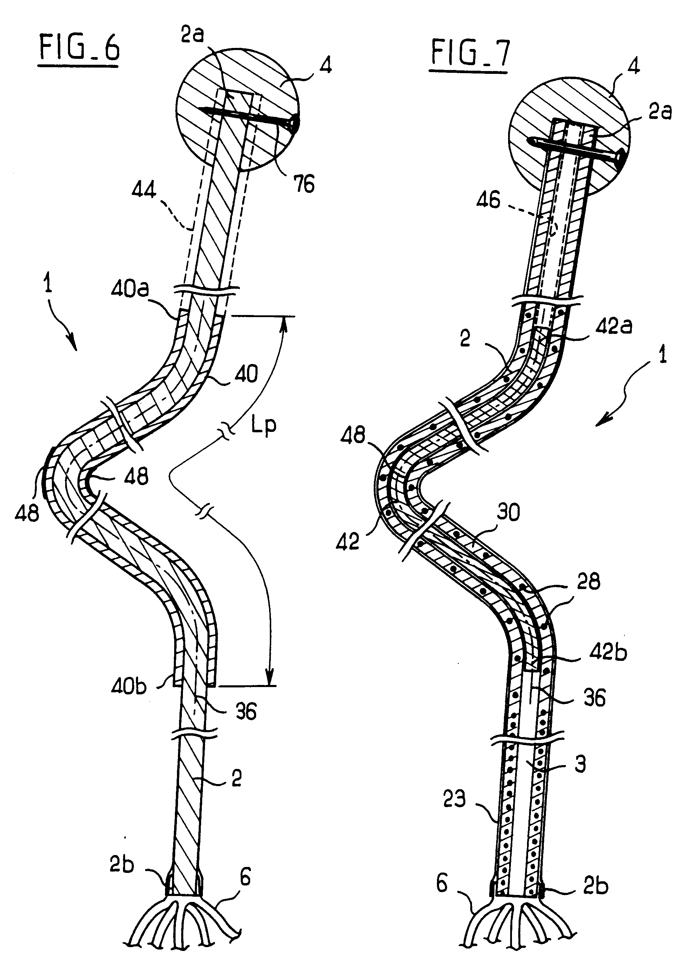

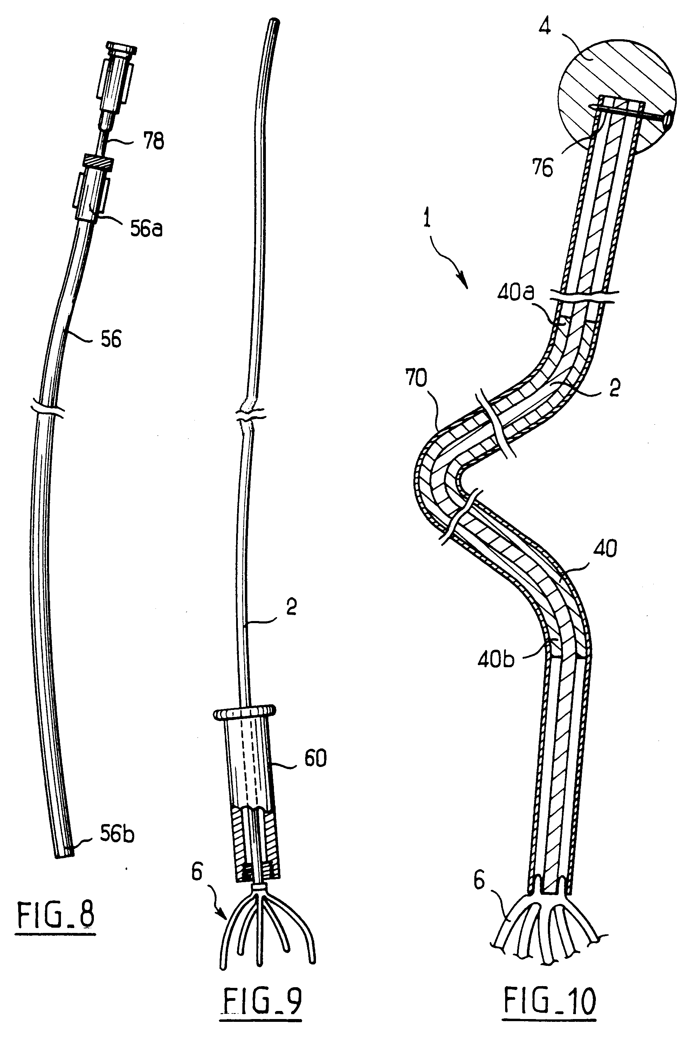

Le dispositif 1 comprend essentiellement une tige souple 2, d'axe 22 colinéaire avec celui du dispositif et de longueur L, avec une extrémité proximale 2a et une extrémité distale opposée 2b. Cette tige, avantageusement entourée d'une fine gaine 23 de protection, par exemple en silicone, pourra être pleine (figure 6, 10) ou présenter un passage intérieur 3 (figures 3, 7).

La tige 2 pourra présenter une rigidité axiale sensiblement constante (figure 6) ou variable (figures 3, 7) sur sa longueur. Elle sera alors plus raide du côté de son extrémité distale, la rigidité allant en diminuant par tronçons, en direction de l'extrémité proximale où la tige est la plus souple. Pour faire varier cette rigidité, on pourra notamment utiliser des tubes coaxiaux 16, 18, de différentes longueurs, avec un ressort métallique hélicoïdal 24 noyé dans la paroi du tube 16, vers son extrémité distale (figure 3), ou encore un unique tube 30 d'épaisseur sensiblement constante dans la paroi duquel est noyé un ressort hélicoïdal 28 dont le "pas" entre spires va en décroissant progressivement (figure 7) depuis l'extrémité distale. Pour plus de détails, on se reportera à la demande française N° 92 13909 du 19/11/1992, correspondant à US-08/152361 du 16/11/1993.

A son extrémité proximale, la tige ou cathéter est ici reliée à une "olive de repérage" 4 classique destinée à permettre de loger la tige entièrement en sous-cutané, cet embout 4 renflé en facilitant le repérage, par palpation à travers la peau (voir EP-A-0521222 ou US-07/731536 du 17/07/1991). Si la tige est creuse, on pourrait même prévoir d'utiliser, en alternative, une chambre implantable de distribution d'un produit traitant, telle que décrite dans la demande française précitée N° 92 13909.

A son extrémité distale, la tige est reliée à un filtre sanguin 6 classique qui présente, en position déployée, une forme générale de corolle conique. Ce filtre comprend ici cinq pattes toutes issues d'une tête commune 7 et dépourvues de tout moyen d'ancrage au vaisseau.

Ces pattes pourront être auto-expansibles pour avoir naturellement tendance à se déployer radialement jusqu'à ce que leur extrémité libre vienne au contact du vaisseau, sans pour autant donc s'y accrocher.

Le filtre, en général métallique, pourra être fixé à la tige par sertissage et/ou collage de sa tête 7.

Ce filtre 6 étant destiné à être implanté à l'intérieur d'un vaisseau sanguin, ici la veine cave inférieure 50, on a constaté qu'il était particulièrement soumis aux fluctuations du flux sanguin ainsi qu'à d'autres contraintes mécaniques telles que celles occasionnées par les mouvements du patient ou encore à la poussée que peut éventuellement exercer sur lui un caillot de sang. Or, de telles contraintes diverses tendent à se propager le long de la tige, celle-ci étant également dans le flux sanguin. L'organe de repérage 4 peut alors être soumis à déplacement, ce qui est peu confortable pour le patient et risque même d'entraîner des complications annexes.

Pour assurer une bonne stabilité du dispositif dans son ensemble, celui-ci présente localement, conformément à l'invention, une zone infléchie ou courbée d'amortissement. Cette zone courbée, ici prévue pour être implantée dans l'oreillette droite du coeur, forme une sorte de "ressort" de maintien de l'unité 1, sensiblement dans la position voulue par le praticien au moment de l'implantation.

Figures 1 et 3, la zone courbée repérée 8, consiste en une portion intermédiaire de la tige elle-même, ici sensiblement en arc plan, avec une extrémité proximale 8a et une extrémité distale 8b.

Pour obtenir cette "préforme" courbe, la tige 2 pourra être constituée par exemple en une matière thermoplastique biocompatible, telle que du polyuréthane ou du polyamide, chauffée à l'endroit de sa portion 8, jusqu'à ramolissement de sa matière. A cette température (de l'ordre du point VICAT) inférieure à celle de fusion, la tige peut être courbée à volonté et conserve, après refroidissement, dans un état de repos non contraint, sensiblement la forme courbée imposée, tout en demeurant flexible sensiblement élastiquement. En alternative, on pourrait aussi réaliser la tige en silicone extrudé, par exemple dans un état semi-réticulé, puis mis dans sa forme courbe dans un moule où la réticulation se termine.

Quoi qu'il en soit, cette zone 8 pourra ensuite être aisément déformée pour s'étendre linéairement suivant l'axe 10 lors de la phase d'introduction de la tige dans l'organisme du patient, tout en formant, une fois la tige libérée, le "ressort" précité de stabilisation, en cas de poussée, voire de traction, exercée sur la tige en particulier par le filtre 6.

A noter que la zone courbée comprend un repère radio-opaque 48, tel une bague métallique, permettant au praticien de pouvoir la repérer dans l'organisme.

Les figures 4 et 5 montrent deux variantes de réalisation de cette zone qui, à la figure 4, se présente sous la forme générale d'un serpentin 12 sensiblement plan, tandis qu'à la figure 5, elle se développe en s'enroulant sensiblement suivant l'axe 22, telle une spirale ou hélice 14 à au moins une spire.

Aux figures 6 et 7, la tige n'a pas été préformée courbée, mais on lui a adjoint une pièce de mise en forme 40, 42, d'axe 36, courbe dans un état de repos et montée glissante avec un faible jeu radial le long de la tige, pour un positionnement de la zone courbée adapté à chaque morphologie. Cette pièce, bien que souple, présente une rigidité supérieure ou égale à celle de la tige.

Sur la figure 6, la pièce en question est un tube flexible 40, tandis qu'à la figure 7, il s'agit d'un cylindre plastique 42, glissé dans le passage 3 de la tige qui est alors creuse.

Ces pièces 40 ou 42, seront de préférence pourvues d'un repère radio-opaque 48 et pourront être réalisées en plastique ou en élastomère avec préformage d'une portion au moins de leur longueur (Lₚ), comme précédemment décrit. Cette portion de maintien de la forme de la tige, avec une extrémité distale (40b, 42b) et une extrémité proximale (40a, 42a), peut être prolongée vers l'extrémité proximale de la tige par une partie 44, 46 non préformée (en pointillés sur les figures 6 et 7).

En relation avec les figures 2, 8 et 9, nous allons à présent décrire une méthode possible d'implantation de l'unité de filtration, par voie percutanée, dans la veine cave inférieure 50 d'un patient H.

Comme décrit dans la demande française N° 92 13909, l'opérateur peut d'abord ménager dans le cou une voie d'accès percutanée VA de la veine jugulaire 58. Il insère ensuite, sur un guide-fil et un dilatateur (non représentés), une gaine introductrice 56 à travers la veine 58, la veine cave supérieure 52 et enfin la veine cave inférieure 50 jusqu'à la zone d'implantation 54 du filtre.

Une fois la gaine tubulaire 56 en position, elle sert de guide pour la mise en place du filtre, lequel est habituellement préconditionné à l'état radialement rapproché de ses pattes dans une sorte de seringue de conditionnement 60 vissée sur l'embout proximal 56a de la gaine qui débouche hors du corps du patient.

L'opérateur fait alors descendre le filtre toujours replié (avec la tige éventuellement temporairement rigidifiée à sa suite) dans la gaine jusqu'à ce que le filtre parvienne à l'extrémité distale 56b de la gaine. Il tire alors vers lui la gaine pour que le filtre soit libéré et s'expanse naturellement.

La gaine remontant, elle libère à son tour, dans l'oreillette droite 72, la zone 8 laquelle se déploie pour prendre sa courbure naturelle et vient en appui, au moins en partie, contre la paroi de l'oreillette. Cette zone déployée sera avantageusement écartée de l'axe 10, de telle sorte que son encombrement radial maximal e soit au moins égal au diamètre de la veine 50 ou 52, cette zone se trouvant alors bloquée dans l'oreillette jusqu'au moment du retrait éventuel du dispositif.

Dans le cas où le dispositif comporte une pièce annexe 40, 42, la gaine 56 est remontée, après la libération du filtre, jusque vers l'oreillette droite (légèrement en dessous). La pièce 40, 42 est alors introduite dans la gaine et est descendue jusqu'à son extrémité 56b éventuellement à l'aide d'un poussoir, tel que 78.

Une fois la pièce annexe arrivée à l'extrémité 56b de la gaine, elle est alors expulsée hors de celle-ci et adopte sa forme courbée à l'intérieur de l'oreillette. L'opérateur peut alors retirer totalement la gaine 56.

Il ne lui reste plus qu'à raccorder l'extrémité proximale de la tige à "l'olive" retenue, via par exemple le poinçon 76, après avoir sectionné à longueur convenable la tige. Il enfouit ensuite le tout dans les tissus et referme la voie d'accès. Lorsque le praticien désire retirer le dispositif, il peut par exemple, après avoir pratiqué une petite incision aux environs de l'élément 4, tirer sur la tige en forçant la zone courbée à s'extraire de l'oreillette 72 en se déformant.

A titre d'exemple, la tige pourra présenter une longueur L comprise entre environ 60 cm et 90 cm et un diamètre extérieur compris entre 2 mm et 5 mm environ. La zone courbée présentera une longueur (dans un état allongé) comprise entre environ 50 mm et 80 mm et de préférence d'environ 70 mm, la distance d séparant les extrémités distales de la zone courbée de celle de la tige pouvant varier de 60 mm à 100 mm environ et être par exemple égale à environ 80 mm.

Bien entendu, l'invention n'est nullement limitée aux modes de réalisation ci-dessus ; ainsi, suivant une variante, on pourra prévoir que la zone courbée soit réalisée au moins en partie en un matériau a mémoire de forme thermique tel que du "NITINOL" (marque déposée, alliage à base de nickel et de titane), reprenant sa forme naturellement et localement, une fois chauffée par le flux sanguin.

Suivant une autre variante illustrée figure 10, on pourrait prévoir une gaine souple 70 entourant la tige 2 (ici pleine) avec un certain jeu. Cette gaine, fixée au filtre à son extrémité distale 70b, aurait une longueur proche de celle de la tige. Elle permettrait le coulissement aisé de la pièce tubulaire 40 entre elle et la tige.

Dans d'autres cas d'implantation de la tige flexible, on pourrait également concevoir que la zone-ressort de la tige soit placée dans une cavité autre que l'oreillette 72 du coeur, voire à l'endroit d'un embranchement de vaisseaux.

## Revendications

**1)** Dispositif à usage médical propre à être introduit dans un conduit d'un corps humain ou animal vivant, le dispositif présentant un axe (10) et comprenant une tige flexible (2) qui présente une extrémité proximale (2a), une extrémité distale (2b) et une longueur (L) suivant l'axe du dispositif, caractérisé en ce qu'il présente localement, sur une partie de la longueur de la tige, au moins une zone-ressort courbée (8, 12, 14, 40, 42) d'amortissement pour la stabilisation axiale du dispositif, ladite zone-ressort étant propre à varier de courbure lors d'un mouvement axial de l'une desdites extrémités de la tige par rapport à l'autre.

**2)** Dispositif selon la revendication 1, caractérisé en ce que ladite zone courbée présente la forme générale d'un arc (8).

**3)** Dispositif selon la revendication 1, caractérisé en ce que ladite zone courbée (14) se développe en s'enroulant sensiblement suivant l'axe du dispositif.

**4)** Dispositif selon la revendication 1, caractérisé en ce que ladite zone courbée se présente sous la forme générale d'un serpentin (12) sensiblement plan.

**5)** Dispositif selon l'une quelconque des revendications 1 à 4, caractérisé en ce que la tige est réalisée en une matière qui conserve d'elle-même la forme qui lui est imposée, ladite tige présentant dans un état de repos, une portion courbée (8, 12, 14) à courbure imposée qui constitue ladite zone de stabilisation.

**6)** Dispositif selon l'une quelconque des revendications 1 à 4, caractérisé en ce qu'il comporte une pièce (40, 42) de maintien de la forme de la tige (2), disposée le long d'une partie de cette tige, ladite pièce présentant, au moins localement, dans un état de repos, une portion courbée à courbure imposée de manière à constituer avec la tige ladite zone courbée.

**7)** Dispositif selon la revendication 6, caractérisé en ce que ladite pièce de maintien est disposée coulissante le long de la tige pour faire varier la position axiale de ladite zone courbée.

**8)** Dispositif selon l'une quelconque des revendications 1 à 7, caractérisé en ce qu'il est propre à être introduit dans un vaisseau sanguin et comprend un filtre sanguin (6) fixé à l'extrémité distale de la tige.

**9)** Dispositif selon la revendication 8, caractérisé en ce que la tige présente une rigidité variable, étant plus raide du côté de son extrémité distale (2b) et plus souple du côté de son extrémité proximale (2a), la rigidité allant en décroissant par tronçons en direction de l'extrémité proximale.

**10)** Dispositif selon l'une quelconque des revendications 1 à 9, caractérisé en ce que la zone courbée présentant une extrémité proximale (8a, 40a, 42a) et une extrémité distale (8b, 40b, 42b), la distance (d) séparant l'extrémité distale de ladite zone courbée et celle de la tige est comprise entre environ 60 mm et 100 mm.

**11)** Dispositif selon l'une quelconque des revendications 6 à 9, caractérisé en ce qu'il comporte une gaine extérieure tubulaire entourant ladite tige avec un certain jeu, et en ce que ladite pièce de maintien est disposée entre cette gaine et la tige.

**12)** Tige (2) flexible, à usage médical présentant:
- un axe (22),
- une extrémité proximale (2a),
- une extrémité distale (2b),
- une longueur (L) suivant son axe,
- et, sur une partie de sa longueur, dans un état de repos, au moins une zone-ressort courbée d'amortissement (8, 12, 14) a courbure déformable, cette zone-ressort étant sensiblement élastiquement déformable en flexion pour reprendre sensiblement sa forme ainsi courbée après qu'on ait poussé ou tiré sur elle suivant l'axe (22) de la tige.

**13)** Pièce à usage médical propre à être montée le long d'une tige à usage médical pour le maintien de la forme de celle-ci, ladite pièce (40, 42) présentant un axe (36) et une longueur (Lₚ) suivant cet axe et, sur une partie au moins de cette longueur, dans un état de repos, une zone-ressort courbée (40, 42) d'amortissement pour la stabilisation axiale de la tige, cette zone présentant une courbure élastiquement déformable en flexion pour reprendre sensiblement sa forme après qu'on ait poussé ou tiré sur elle ou sur la tige suivant l'axe de la pièce.
